# EUROPEAN PATENT APPLICATION

(11) **EP 1 525 891 A1**
(43) Date of publication of application: **27.04.2005**
(21) Application number: 04077868.0
(22) Date of filing: 19.10.2004
(51) Int. Cl.: A61L 2/20, A01M 1/20, B27K 3/00

(54) **Device for treating products by means of a gas**

(30) Priority: 21.10.2003 BE 200300556
(71) Applicant: Desclean Belgie, 2610 Wilrijk (BE); Van Duyse, Koen, 2830 Blaasveld (BE)
(72) Inventor: VAN DUYSE, Koen (Prosper Alfonsine), 2830 Blaasveld (BE); WILLIAME, Eddy (Julia Emile), 2610 Antwerpen (WILRIJK) (BE)
(74) Representative: Donné, Eddy

(57) **Abstract**

Device for treating products by means of a gas, whereby it is mainly composed of a dome (3) which can be provided over the products (13) to be treated, means which make it possible to seal the lower edge of the dome (3) in a gas-tight manner when it is provided over the products (13) to be treated, and means for spreading a gas in the dome (3).

## Description

The present invention concerns a device for treating products by means of a gas.

More particularly, it concerns a device for treating transport goods, for example wood, that have to be treated against possible diseases and plagues, in order to prevent such diseases and plagues from being introduced in the country of destination. For this reason, these goods are treated with a certain quantity of gas, for example a poisonous gas for exterminating bacteria, insects and the like.

To this end, the above-mentioned goods must be confined in a gas-tight room in one way or another in order to put gas on them.

It is known that use is made to this end of what is called clamp gassing, whereby the load of goods is placed on what is called a flat bed container and is covered by a tarpaulin, to subsequently blow gas under the tarpaulin.

A disadvantage of this known clamp gassing is that covering the goods in a gas-tight manner under a tarpaulin is very difficult to realize, among others due to the shape of the container and of the bottom of the container, and due to the fact that the bottom of a flat bed container is not gas-tight itself.

Another disadvantage is that, when using a tarpaulin, there will always remain openings through which the gas can escape. This results in an excessive gas consumption and, consequently, in high treatment costs. Moreover, this is environmentally damaging.

Another disadvantage is that such flat bed containers are usually situated in an open territory, for example in river and sea terminals, where they are exposed to every kind of weather and where, consequently, manipulating the tarpaulins is not without danger for the workers having to provide the tarpaulins.

Also bunkers are already known in which the goods to be treated are placed and are treated with the gas concerned.

A disadvantage of these bunkers is their very high cost price and the fact that the construction is not mobile.

Another disadvantage of such bunkers is that their volume is too large and that, consequently, when the bunker is only partly filled with goods, there is much gas consumption. This results in a higher cost price of the treatment process and, especially when the gases are poisonous, to environmental damage.

The present invention aims to remedy the above-mentioned and other disadvantages by providing a mobile and safe device which is easy to use and which leads to an ecologically sound treatment of the transport goods, characterized by a low cost price of the treatment process.

To this end, the invention concerns a device for treating products by means of a gas, whereby it is mainly composed of a dome which can be provided over the products to be treated before use, means which make it possible to seal the lower edge of the dome in a gas-tight manner after it has been put over the products to be treated, and means for spreading a gas in the dome.

In a practical embodiment, this dome consists of a self-supporting construction formed of connecting side walls and an upper wall and which is open at the bottom, such that it can be put over the products to be treated, and which is provided with crane hooks for lifting and moving the dome.

According to a preferred characteristic of the invention, the dome is provided with an exhaust for the gas. This exhaust may for example consist of a fan placed opposite to an opening in one of the walls.

According to another preferred characteristic of the invention, the means for sealing the lower edge of the dome in a gas-tight manner consist of a strip, made of an elastic material, which is provided under the above-mentioned lower edge of the dome.

In the most preferred embodiment, the device for treating goods by means of a gas is provided with a loose bottom whose dimensions are practically equal to the dimensions of the perimeter of the above-mentioned lower edge of the dome, whereby this loose bottom is preferably formed of an elastic and gas-tight mat, for example a synthetic rubber.

The dome is preferably designed such that means are provided, for example in de shape of inflatable elements, which make it possible to reduce the space which is confined by the dome. In this manner, the use of gas, whether or not poisonous, is strongly reduced, which will lower the cost price of a gas treatment.

In order to better explain the characteristics of to the invention, the following preferred embodiments are described as an example only without being limitative in any way, with reference to the accompanying drawings, in which:
figure 1 schematically represents a device according to the invention in perspective;
figure 2 represents a section according to line II-II in figure 1;
figures 3 to 8 represent how the device is used in steps;
figures 9 to 11 represent variants of the device during successive steps of use.

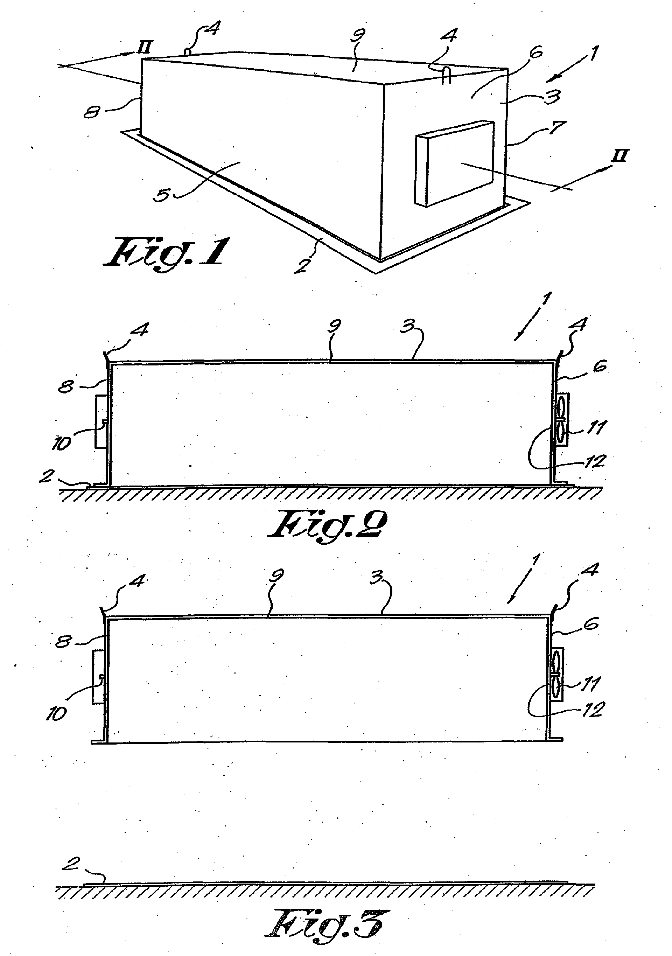

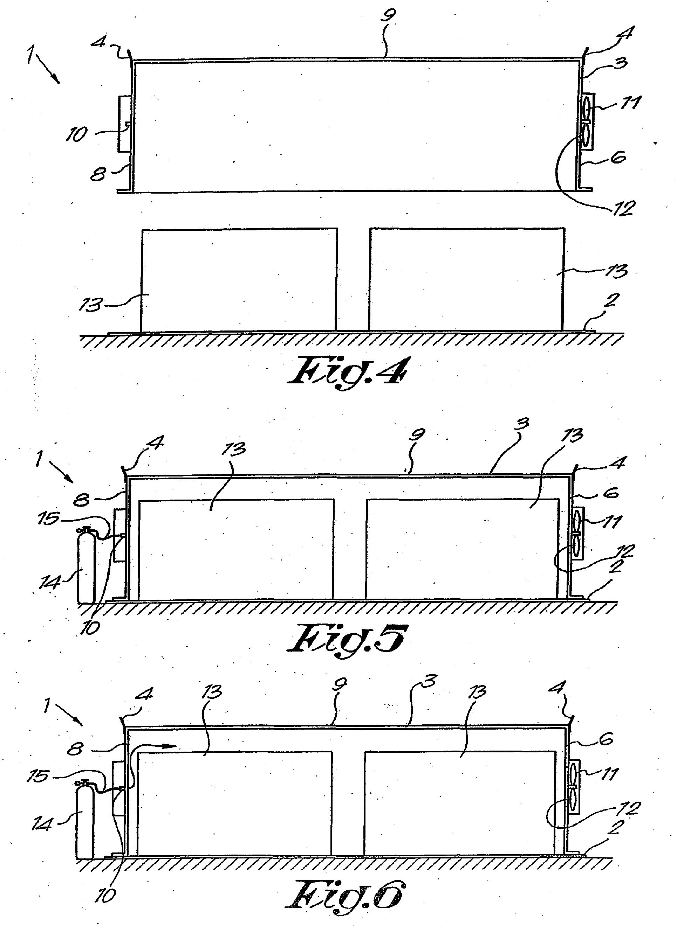

As represented in figures 1 and 2, the device 1 according to the invention mainly consists of an elastic and gas-tight mat 2, upon which is put a dome 3 provided with crane hooks 4.

In this example, the above-mentioned dome 3 consists of a sort of bottomless container, formed of four connecting side walls 5, 6, 7 and 8 and a sealing upper wall 9.

The inside of the above-mentioned dome 3 must be gas-resistant. The dome 3 must be as much gas-tight as possible.

The device 1 comprises means for spreading a gas in the dome 3, in this case in the shape of a connection 10 for a gas supply, which connection 10 is for example provided in the side wall 8.

Moreover, the device 1 is provided with an exhaust for gases, for example in the shape of a fan 11 provided opposite to an opening 12 in an opposite side wall 6.

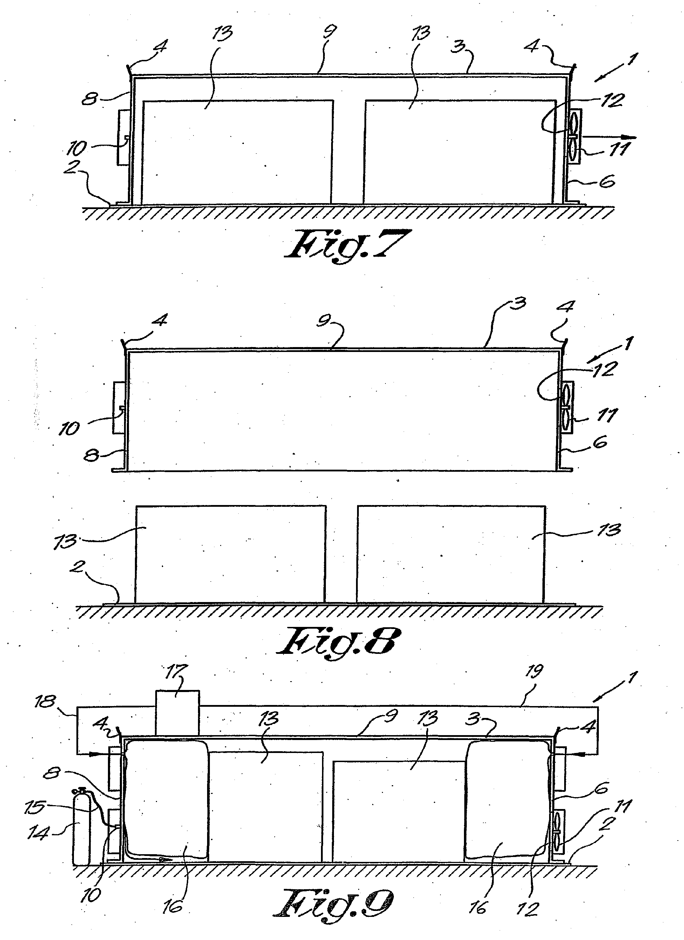

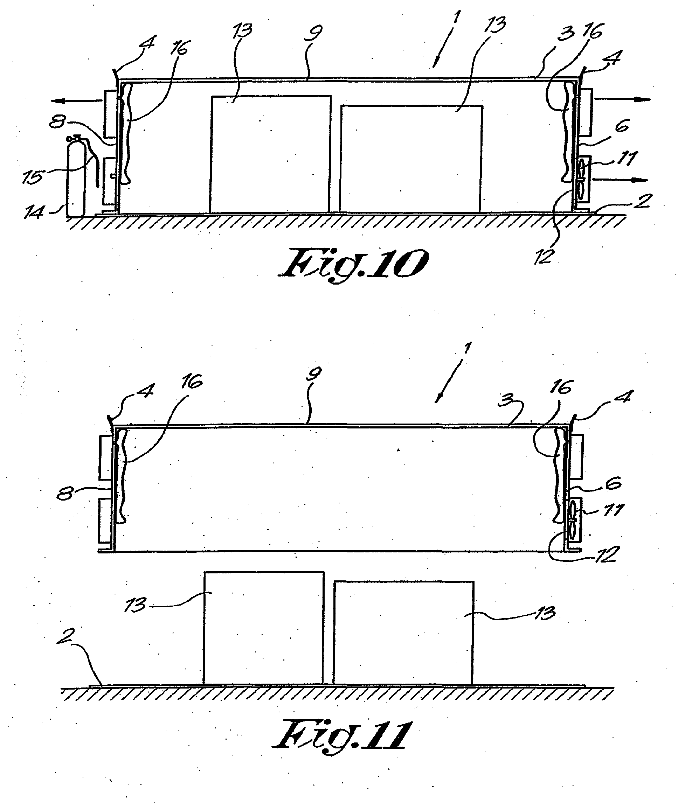

The use of the above-mentioned device 1 according to the invention is very simple and as follows, and it is illustrated by way of example of the figures 3 to 8.

Figure 3 shows how the dome 3 is lifted by the crane hooks 4, by means of a hoist which is not represented in the figures.

In figure 4, the products to be treated 13 are placed on the elastic and gas-tight mat 2.

Next, as represented in figure 5, the dome 3 is lowered over the products 13 and onto the mat 2, and a gas supply in the shape of for example a gas cylinder 14 is connected to the connection 10 by means of the supply line 15, after which gas can be spread from the gas cylinder 14 into the space of the dome 3 in order to treat the products 13.

After the treatment, as represented in figure 7, the gas cylinder 14 is disconnected again and the fan 11 is activated to remove the gas from the dome 3 in this manner.

Finally, figure 8 shows how, when the gas has been removed from the dome 3, the dome 3 can be lifted again and the products 13 can be picked up further so as to remove them for further storage or treatment.

It is possible to provide the dome 3 with a thermal isolation, for example a thermal coating, so that the temperature in the dome 3 will not rise too much when it is exposed to sun light, for example in hot regions.

In cold regions, the thermal coating is meant to keep the temperature in the dome 3 at a certain level while gas is being sprayed in, with a minimum of energy consumption. For, treating products 13 by means of a gas should be done above a certain minimum temperature. For spraying gases on wood, this minimum temperature amounts to 5 degrees Celsius. However, depending on the gas to be used and on the products to be treated 13, the value of this minimum temperature may be higher. Thanks to the thermal coating, additional heating is restricted or even made redundant.

In a practical embodiment, one or several heating elements can possibly be provided in the dome 3 to make sure that the above-mentioned minimum temperature can be reached at all times.

Figure 9 shows a variant of the device according to the invention whereby, in this case, inflatable elements 16 are provided in the dome 3, and whereby also a compressor 17 is provided which is connected to the inflatable elements 16 by means of two pressure pipes.

The use of the above-mentioned variant according to the invention is represented in figures 9 to 11. In this case as well, the dome 3 is placed over the products to be treated 13 and onto the mat 2, and additionally, the inflatable elements 16 are inflated by means of the compressor 17, such that the free space between the products to be treated 13 and the dome 3 is reduced, as a result of which less gas will have to be spread in this free space.

Next, after the products 13 have been treated, the gases are exhausted and the inflatable elements 16 are deflated. Finally, the dome 3 can be removed again, and the products 13 can be further manipulated.

The present invention is by no means limited to the embodiments given as an example and represented in the accompanying drawings; on the contrary, such a device for treating products by means of a gas according to the invention can be made in all sorts of shapes and dimensions while still remaining within the scope of the invention.

## Claims

1. Device for treating products by means of a gas, **characterized in that** it is mainly composed of a dome (3) which can be provided over the products (13) to be treated, means which make it possible to seal the lower edge of the dome (3) in a gas-tight manner when it is provided over the products (13) to be treated, and means for spreading a gas in the dome (3).

2. Device according to claim 1, **characterized in that** the above-mentioned dome (3) is formed of a self-supporting construction which is formed of connecting side walls (5, 6, 7 and 8) and a sealing upper wall (9), and which is provided with an opening at the bottom.

3. Device according to claim 1, **characterized in that** the dome (3) is provided with an exhaust for the gas.

4. Device according to claim 1, **characterized in that** the dome (3) is provided with crane hooks (4) which make it possible to lift the dome (3).

5. Device according to claim 1, **characterized in that** the walls (5, 6, 7, 8 and 9) of the dome (3) are provided with a thermal coating.

6. Device according to claim 1, **characterized in that** the means which make it possible to seal the lower edge of the dome (3) in a gas-tight manner are formed of a strip, made of an elastic material, which is provided under the above-mentioned lower edge of the dome (3).

7. Device according to claim 1, **characterized in that** it is provided with a loose bottom whose dimensions are at least practically equal to the dimensions of the perimeter of the above-mentioned lower edge of the dome (3).

8. Device according to claim 7, **characterized in that** the above-mentioned loose bottom is formed of an elastic and gas-tight mat (2).

9. Device according to claim 1, **characterized in that** it is provided with means which make it possible to reduce the space which is confined by the dome (3).

10. Device according to claim 9, **characterized in that** the above-mentioned means for reducing the space of the dome (3) are formed of inflatable elements (16).
